# EUROPEAN PATENT SPECIFICATION

(11) **EP 1 500 372 B1**
(45) Date of publication and mention of the grant of the patent: **24.03.2010**
(21) Application number: 04025695.0
(22) Date of filing: 28.03.1995
(51) Int. Cl.: A61B 17/064

(54) **Apparatus for spinal fixation**
Einrichtung zur Wirbelsäulenfixation
Appareil de fixation vertébrale

(30) Priority: 28.03.1994 US 219626
(43) Date of publication of application: 26.01.2005
(62) Divisional of application: 01128856.0
(73) Proprietor: Warsaw Orthopedic, Inc., Warsaw, IN 46581 (US)
(72) Inventor: Michelson, Gary Karlin, Los Angeles, CA 90049 (US)
(74) Representative: Viering, Jentschura & Partner

(56) References cited:
- EP-A- 0 356 112
- EP-A- 0 421 485
- WO-A-93/10725
- DE-B- 2 649 042
- US-A- 4 401 112
- US-A- 5 015 247
- US-A- 5 092 893

## Description

### BACKGROUND OF THE INVENTION

### Field of the Invention

The invention relates to an apparatus for linking multiple spinal implants. Accordingly this invention generally relates to surgical interbody fixation devices and in particular to a surgically implantable device for the stabilization of adjacent vertebrae of the human spine undergoing spinal arthrodesis and for the prevention of the dislodgement of spinal fusion implants used in the fusion process.

### Description of the Related Art

When a segment of the human spine degenerates, or otherwise becomes diseased, it may become necessary to surgically remove the affected disc of that segment, and to replace it with bone for the purpose of obtaining a spinal fusion by which to restore more normal, pre-morbid, spatial relations, and to provide for enhanced stability across that segment. Performing such surgery of the spine from an anterior (front) approach offers the great advantage of avoiding the spinal cord, dural sac; and nerve roots. Unfortunately, in entering the disc space anteriorly a very important band-like structure called the anterior longitudinal ligament, is violated. This structure physiologically acts as a significant restraint resisting the anterior displacement of the disc itself and acting as a tension band binding the front portions of the vertebrae so as to limit spinal hyperextension.

Historically, various devices have been utilized in an attempt to compensate for the loss of this important stabilizing structure. These devices have assumed the form of blocks, bars, cables, or some combination thereof, and are bound to the vertebrae by screws, staples, bolts, or some combination thereof. The earliest teachings are of a metal plate attached to adjacent vertebrae with wood-type screws. Dwyer teaches the use of a staple-screw combination. Brantigan U.S. Patent No. 4,743,256 issued on May 10, 1988, teaches the use of a block inserted to replace the disc, affixed to a plate then screwed to the vertebrae above and below. Raezian U.S. Patent No. 4,401,112 issued on August 30, 1993, teaches the use of a turnbuckle affixed to an elongated staple such that at least one entire vertebral body is removed, the turnbuckle portion is placed within the spine, and the staple extends both above and below the turnbuckle and engages the adjacent vertebrae to the one removed.

Unfortunately, both staples and screws have quite predictably demonstrated the propensity to back out from the vertebrae. This is quite understandable as any motion, either micro or macro, tends to stress the interface of the metallic implant to the bone, and in doing so causes the bone to relieve the high:stress upon it by resorbing and moving away from the metal. This entropic change is universally from the more tightened and thus well-fixated state, to the less tightened and less fixated state. For a staple, this is specifically from the more compressed and engaged state, to the less compressed and disengaged state. Similarly, screws in such a dynamic system loosen and back out.

The potential consequences of such loosening and consequent backing out of the hardware from the anterior aspect of the vertebral column may easily be catastrophic. Because of the proximity of the great vessels, aortic erosions and perforations of the vena cava and iliac vessels have usually occurred with unfortunate regularity and have usually resulted in dearth.

Therefore, the need exists for a device which is effective in restoring stability to a segment of the spine such as, but not limited to, the anterior aspect of the human spine and which will without danger remain permanently fixated once applied.

US-A-5015247 discloses apparatus comprising first and second spinal implants as in the preamble to appended claim 1.

### SUMMARY OF THE INVENTION

To this end, the invention provides an apparatus for linking multiple spinal implants, having the features of claim 1. Further embodiments of the invention are described in the dependent claims.

The present invention is broadly applicable to the anterior, posterior and lateral aspects of the spinal column, be it the cervical, thoracic or lumbar area. In particular, the use of a staple member spanning the disc space and engaging the adjacent vertebrae which is applied to the anterior aspect of the spine is of great utility in restraining those vertebral bodies from moving apart as the spine is extended and thus is effective in replacing the anterior longitudinal ligament of the patient.

The spinal fixation device of the present invention may be coated with materials to promote bone fusion and thus promote the incorporation and ultimate entombment of the spinal fixation device into the bone fusion mass. The use of a bone fusion promoting material results in a speedier vertebra to vertebra fusion as bone may grow along the coated spinal fixation device bridging the two vertebrae so that the spinal fixation device acts as a trellis and supplies essential chemical elements to facilitate the bone fusion process.

An advantage of the spinal fixation device of the present invention is that it may be used as an anchor such that a multiplicity of spinal fixation devices may then be interconnected via a cable, rod, bar, or plate, so as to achieve or maintain a multi-segmental spinal alignment.

Alternatively, the spinal fixation device of the' present invention could be made of resorbable materials, such as bio-compatible resorbable plastics, that resorb at an appropriate rate such that once the spinal fixation device is no longer needed (i.e. when spinal fusion is complete) the body would resorb the spinal fixation device. The spinal fixation device could be only in part resorbable, for example, such that projections of the staple member would be non-resorbable and would remain incarcerated in the vertebrae and sealed off once the resorbable portion of the staple is resorbed by the body.

As a further alternative, the spinal fixation device could be made wholly of in part of ceramic and more particularly made of or coated with a ceramic such as hydroxyapatite that would actively participate in the fusion process.

Hence, the present invention provides an apparatus that blocks the rotation of the intervertebral spinal fusion implants in such a way as to be incapable of rotation thereby preventing the spinal fusion implant from backing out.

The present invention is broadly applicable to the anterior aspect of the spinal column, be it the cervical,thoracic or lumbar area;

In the present invention, the spinal fixation device which may be applied longitudinally at any point about the circumference of the anterior aspect of the spine.

The spinal fixation device stabilizes the surgically implanted spinal fusion implants and works in connection with the spinal fusion implant to prevent disengagement of either.

The spinal fixation device may serve as an anchor, such that a multiplicity of these anchors may then be interconnected via a cable, rod, bar, or plate, so as to achieve or maintain a multi-segmental spinal alignment. These and other aspects of the present invention will become apparent from a review of the accompanying drawings and the detailed description of the drawings.

### BRIEF DESCRIPTION OF THE DRAWINGS

The spinal fixation device, locking screw, securing means, drill template instrument according to Figs. 1, 5-12, 13A-13F, 14, 15A-15C and 18 do not form part of the invention.
Figure 1 is a perspective side view of a segment of the spinal column having two spinal fusion implants shown partially in hidden line inserted across the disc space between two adjacent vertebrae with each spinal fusion implant having a spinal fixation device shown partially in hidden line secured thereto, spanning across the disc space and inserted into the vertebrae.
Figure 2 is a perspective side view of a segment of the spinal column having two spinal fusion implants inserted across the disc space between two adjacent vertebrae.
Figure 3 is an elevational side view of a cylindrical threaded spinal fusion implant.
Figure 4 is an end view of the cylindrical threaded spinal fusion implant along lines 4--4 of Figure 3.
Figure 5 is a perspective side view of a segment of the spinal column having two non-threaded spinal fusion implants with external ratchetings, shown in hidden line, inserted across the disc space between two adjacent vertebrae with each spinal fusion implant having a spinal fixation device, shown partially in hidden line, coupled thereto, spanning across the disc space and inserted into the vertebrae.
Figure 6 is a perspective side view of a segment of the spinal column having two spinal fusion implants having truncated sides with external ratchetings shown in hidden line inserted across the disc space between two adjacent vertebrae with each spinal fusion implant having a spinal fixation device shown partially in hidden line coupled thereto, spanning across the disc space and inserted into the vertebrae.
Figure 7 is a perspective side view of a segment of the spinal column having two spinal fusion implants having a knurled external surface shown in hidden line inserted across the disc space between two adjacent vertebrae with each spinal fusion implant having a spinal fixation device shown partially in hidden line coupled thereto, spanning across the disc space and inserted into the vertebrae.
Figure 8 is a top plan view of the spinal fixation device.
Figure 9 is a side view of the spinal fixation device along lines 9--9 of Figure 8.
Figure 10 is a cross sectional view taken along lines 10--10 of Figure 8 showing the top member of the spinal fixation device.
Figure 11 is an enlarged fragmentary perspective side view of a projection of the spinal fixation device taken along line 11 of Figure 9.
Figure 12 is a cross sectional view of the spinal fixation device inserted into the vertebrae and secured to the spinal fusion implant with the arrows showing the forces exerted, the rotational axis and the longitudinal axis of the spinal fusion implant.
Figure 13A is a cross sectional view along line 13--13 of Figure 9 of the preferred embodiment of the projections.
Figures 13B, 13C, 13D, 13E, and 13F are cross sectional views taken along line 13--13 of Figure 9 showing alternative embodiments of the projections of the spinal fixation device.
Figure 14 is an enlarged elevational side view of the locking screw used to secure the spinal fixation device of to a spinal fusion implant.
Figure 15A is a cross sectional view of a securing means for locking the locking screw.
Figure 15B is a cross sectional view of a first alternative embodiment of the securing means for locking the locking screw.
Figure 15C is a cross sectional view of a second alternative embodiment of the securing means for locking the locking screw.
Figure 16A is a perspective side view of the instrumentation used for driving the spinal fixation device into the vertebrae.
Figure 16B is a perspective side view of a first alternative embodiment of the instrumentation used for driving the spinal fixation device into the vertebrae.
Figure 17A is a perspective side view of an alignment rod used to align the spinal fixation device.
Figure 17B is a perspective side view of an alternative embodiment of the alignment rod having splines used to align the spinal fixation device.
Figure 18 is a front perspective view of the drill template instrument.
Figure 19 is a perspective side view of the alignment rod attached to a spinal fusion implant inserted in the disc space between two adjacent vertebrae.
Figure 20 illustrates the step of drilling guide holes in the vertebrae adjacent to the spinal fusion implant with the drill template instrument of Figure 18.
Figure 21 illustrates a step of the method of inserting the spinal fixation device with the alignment rod attached to the spinal fusion implant and the spinal fixation device placed on the driver instrumentation.
Figure 22 illustrates a step of the short method of inserting the spinal fixation device with the driver instrument engaging the splined alignment rod and a hammer for applying an impaction force and driving the driver instrument.
Figure 22A is an enlarged fragmentary view of a projection being inserted into an insertion hole drilled within a vertebra shown in cross section taken along line 22A of Figure 21.
Figure 23 illustrates another step of the method of inserting the spinal fixation device in which the spinal fixation device has been driven into the vertebrae and the driver instrumentation has been removed.
Figure 24 illustrates another step of the method of inserting the spinal fixation device with the splined alignment rod being removed from the spinal fusion implant and the locking screw being inserted and secured the spinal fixation device to the spinal fusion implant.

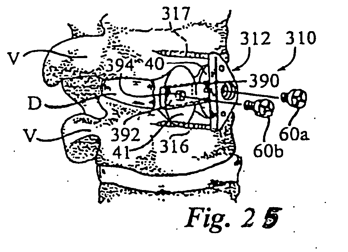

Figure 25 is a perspective side view of an apparatus including a spinal fixation device of the present invention coupled to two spinal fusion implants and inserted in adjacent vertebrae of the spinal column.

### DETAILED DESCRIPTION OF THE DRAWINGS

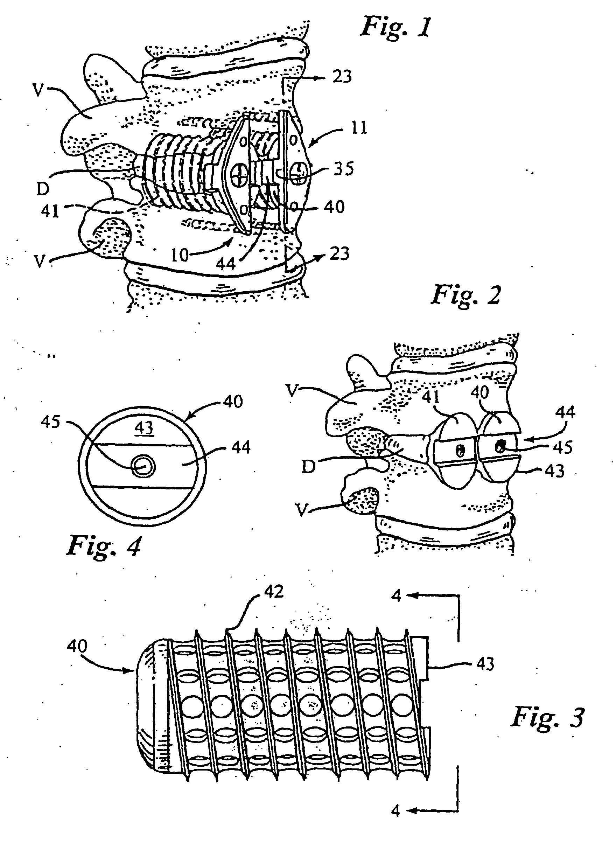

Referring to Figure 1 and 2,' two identical spinal fixation devices, each being generally referred to by the numerals 10 and 11, respectively, are shown inserted into two vertebrae V adjacent to a disc D of a segment of the human spine. Each spinal fixation device 10 and 11 is shown coupled to identical spinal fusion implants 40 and 41 that have been surgically implanted in the disc space between adjacent vertebrae V. In this manner, the spinal fixation devices 10 and 11 stabilize a segment of the spine, prevent the dislodgement of the spinal fusion implant 40, and remain permanently fixated to the spine once applied. The spinal fixation devices 10 and 11 are identical such that the description of one is equally applicable to the other. Thus, the description that follows will be directed to spinal fixation device 10.

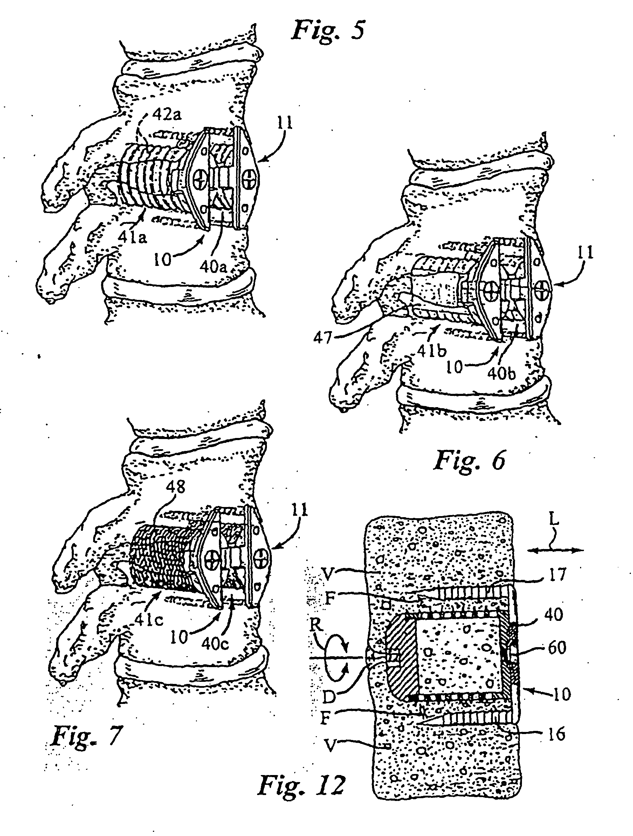

Referring to Figures 3-4, the spinal fusion implant 40 such as, but not limited to, the spinal fusion implant described by Michelson, U.S. Patent No. 5,015,247 issued on May 14, 1991, is shown. The spinal fusion implant 40 is cylindrical in shape and has external threads 42 at its outer perimeter for engaging the bone of the vertebrae V adjacent to the disc D. The spinal fusion implant 40 has an insertion end 43 having a depression 44 and a threaded aperture 45 for engaging a portion of the spinal fixation device 10 and also for engaging a portion of an instrument used to insert the spinal fixation device 10 into the vertebrae V. Referring to Figures 5-7, it is appreciated that the spinal fixation devices 10 and 11 are not limited in use with a threaded spinal fusion implant 40 and 41, but may be used with different types of spinal fusion implants. For example, the spinal fixation devices 10 and 11 may be coupled to spinal fusion implants 40a and 41a, respectively, each having external ratchetings 42a instead of external threads 42 as shown in Figure 5. Alternatively, the spinal fixation devices 10 and 11 may be coupled to spinal fusion implants 40b and 41b, respectively, each having a partially cylindrical shape with at least one truncated side 47 as shown in Figure 6. As a further alternative, the spinal fixation devices 10 and 11 may be coupled to spinal fusion implants 40c and 41c, respectively, each having a knurled external surface 48 as shown in Figure 7. It is also appreciated that the spinal fixation devices may be used with a variety of other bone fusion implants without departing from the scope of the present invention.

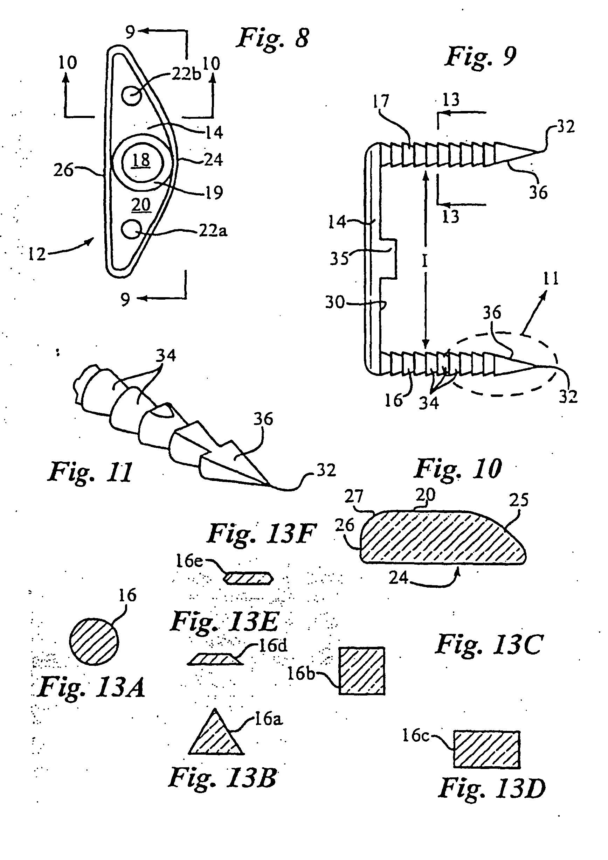

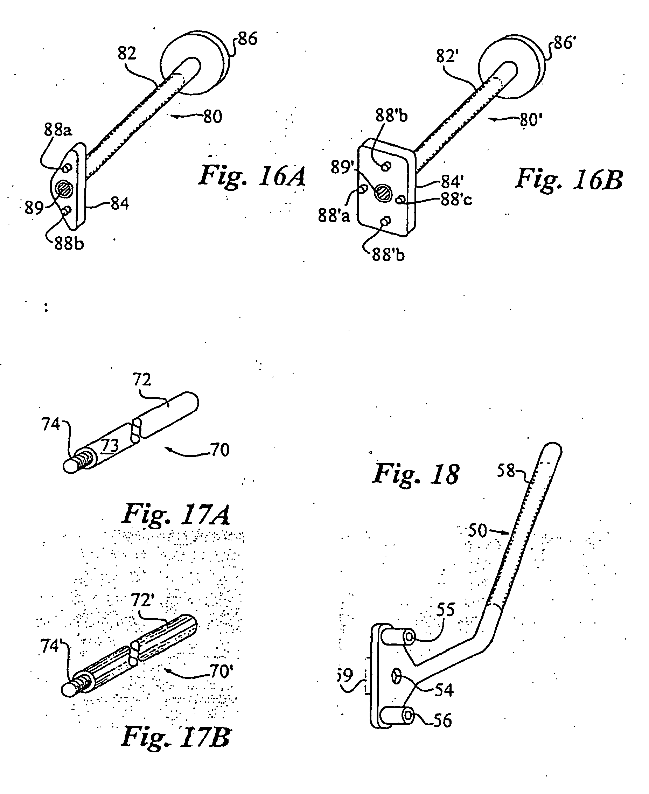

Referring to Figures 8-9, the spinal fixation device 10 comprises a staple member 12 having a substantially planar top member 14 which is of sufficient length to span one intervertebral disc D and to engage, via a plurality of essentially perpendicular extending projections 16 and 17, the vertebrae V adjacent to that disc D. The top member 14 has a central opening 18 within a concentric, countersunk recess 19 for receiving therethrough a screw or similar coupling means for coupling the spinal fixation device 10 to the spinal fusion implant 40. The top member 14 has an upper surface 20 having a pair of openings 22a and 22b for receiving the posts 88a and 88b of a driving instrument 80 which is described in greater detail below in reference to Figures 16A and 16B. Referring to Figure 10, a cross sectional view of the top member 14 is shown. In the preferred embodiment, the top member 14 is generally triangularly shaped and is radiused along curved side 24 and straight side 26. The curved side 24 of the top member 14 is radiused at its upper edge 25 and at the upper edge 27 of straight side 26 to conform to the external curvature of the vertebrae V. In this manner, smooth surfaces are created at the upper edges 25 and 27 of the top member 14 that are contoured to the shape of the external curvature of the vertebrae V when the staple member 12 is in place. The smooth contoured surface of the upper edges 25 and 27 of the top member 14 prevent aortic erosions and perforations of the vessels proximate the vertebral column such as the vena cava and the iliac vessels which might otherwise result from friction. In the preferred embodiment of the spinal fixation device 10, the top member 14 has a width ranging from 6.0 mm to 28.0 mm, with 10.0 mm being the preferred width, and having a thickness in the range of 2.0 mm to 4.0 mm, with 3.0 mm being the preferred thickness. The staple member 12 is made of material appropriate for human surgical implantation including all surgically appropriate metals such as but not limited to, titanium, titanium alloy, chrome molybidium alloys, stainless steel; or non-metallic materials including permanent or resorbable substances or composites, carbon fiber materials, resins, plastics, ceramics or others.

Further, the staple member 12 may be treated with, or even composed of, materials known to participate in or promote in the fusion process or bone growth. The spinal fixation device 10 may be coated with materials to promote bone fusion and thus promote the incorporation and ultimate entombment of the spinal fixation device 10 into the bone fusion mass. The use of a bone fusion promoting material such as, but not limited to hydroxyapatite, hydroxyapatite tricalcium phosphate or bone morphogenic protein, results in a speedier vertebra V to vertebra V fusion as bone may grow along the coated spinal fixation device 10 bridging the two vertebrae V so that the spinal fixation device 10 acts as a trellis and supplies essential chemical elements to facilitate the bone fusion process.

Referring again to Figure 9, the projections 16 and 17 are positioned at opposite ends of the top member 14 and depend downwardly and extend perpendicularly from the bottom surface 30 of the top member 14. The projections 16 and 17 each terminate in a distal end 32 that is pointed and sharpened to facilitate the insertion of the projections 16 and 17 into the vertebrae V.

The staple member 12 is most effective when the interprojection distance I between projections 16 and 17 is at least 4.0 mm and preferably 6.0 mm greater than the diameter of the particular spinal fusion implant 40 for which the spinal fixation device 10 is being used so that at least 2.0 mm and preferably 3.0 mm of bone from the vertebrae V will be present between the spinal fusion implant 40 and each of the projections 16 and 17. Typically, intervertebral spinal fusion implants have a diameter that ranges from 12.0 mm to 28.0 mm, therefore, the interprojection distance I typically will range from 18.0 mm to 34.0 mm for most applications.

In the preferred embodiment, the projections 16 and 17 comprise a series of segmented and ratcheted portions 34. The segmented and ratcheted portions 34 provide for a "one way" insertion of the staple member 12 to prevent the backing-out of the projections 16 and 17 once they are inserted into the bone of the vertebrae V. In the preferred embodiment, each segmented and ratcheted portion 34 of the projections 16 and 17 is conical in shape and the diameter of each segmented and ratcheted portion 34 increases in the direction from the distal end 32 toward the top member 14 so that the projections 16 and 17 resemble a stack of cones. The segmented and ratcheted portions 34 are spaced approximately 2.0 mm to 4.0 mm apart, with 3.0 mm being the preferred distance between each segmented and ratcheted portion 34. Referring to Figure 11-12, in the preferred embodiment of the spinal fixation device 10, in order to further facilitate the insertion of the projections 16 and 17 into the vertebrae V, the distal end 32 of each projection 16 has an eccentric, incline-planed inner surface 36 as shown in Figure 11. The eccentric, incline-planed inner surface 36 of each of the projections 16 and 17 create a force F which pushes the bone of the vertebrae V toward the spinal fusion implant 40 as the staple member 12 is inserted into each of the vertebrae V as shown in Figure 12.

Referring to Figures 13A-13F, in the preferred embodiment of the spinal fixation device 10, the projections 16 and 17 are cylindrical in shape having a circular cross section as shown for projection 16 in Figure 13A. Alternatively, the projection 16a may have a triangular cross section as shown in Figure 13B; the projection 16b may have a square cross section as shown in Figure 13C; the projection 16c may have a rectangular cross section as shown in Figure 13D; the projection 16d may have a trapezoidal cross section as shown in Figure 13E; or the projection 16e may have a cross section with a configuration as shown in Figure 13F.

In the preferred embodiment, the projections 16 and 17 each have a diameter of approximately 2.0 mm to 4.0 mm, with 3.0 mm being the preferred diameter at the widest point. The projection 16 and 17 each have a length ranging from 16.0 mm to 28.0 mm, with 22.0 mm being the preferred length when the spinal fixation device 10 is implanted in the direction of the anterior aspect of the vertebra V to the posterior aspect of the vertebrae V. Alternatively, it is appreciated that the projections 16 and 17 each could have a longer length depending on the diameter of the vertebrae V in which the projections 16 and 17 are implanted.

Referring again to Figure 9, the top member 14 of the staple member 12 has a central bar 35 extending from the center of its bottom surface 30, for interdigitating and mating to an already implanted intervertebral spinal fusion implant 40. In the preferred embodiment the central bar 35 has a thickness in the range of 0.5 mm to 1.5 mm, with 0.5 mm being the preferred thickness.

Referring to Figure 1, the central bar 35 is configured so that it complements and engages the depression 44 at the insertion end 43 of the spinal fusion implant 40. Once engaged to the depression 44, the bar 35 interdigitates with the depression 44 of the spinal fusion implant 40 to lock and prevent the rotation of the spinal fusion implant 40.

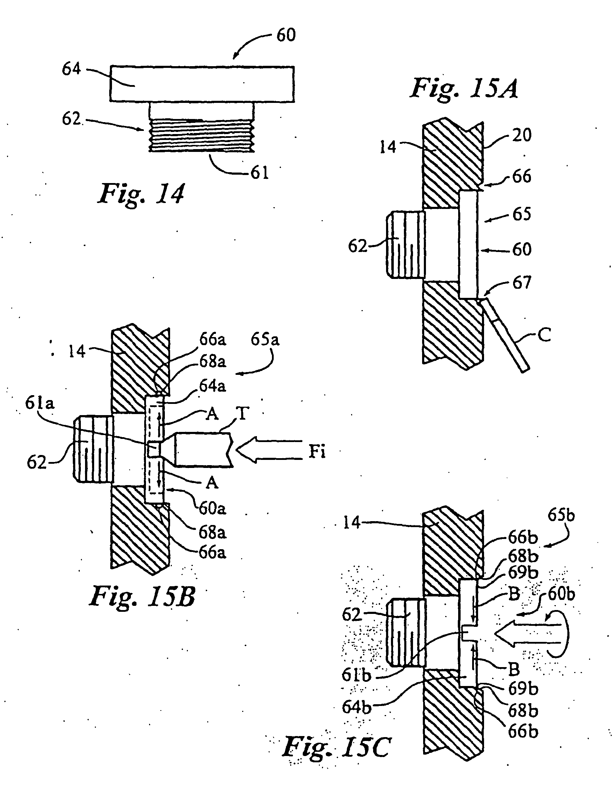

Referring to Figure 14, in the preferred embodiment, the staple member 12 is secured to the spinal fusion implant 40 by a screw 60 having threaded end 61 with a locking thread pattern 62 and screw head 64. The locking thread pattern 62 has a reduced pitch at the bottom of the threaded end 61 such that the screw 60 is self-locking. However, it is appreciated that the threaded pattern 62 may be any of the means for locking a screw well known by those skilled in the art.

Referring to Figures 2 and 8, the threaded end 61 of the screw 60 passes through the central opening 18 of the top member 14 and the threaded pattern 62 threads into the threaded aperture 45 of the spinal fusion implant 40. The screw head 64 fits within the countersunk recess 19 of the top member 14 such that the screw head 64 is at or below the plane of the upper surface 20 of the top member 14. In the preferred embodiment, the central opening 18 has a diameter ranging from 4.5 mm to 5.5 mm, with 5.0 mm being the preferred diameter. The countersunk recess 19 has a diameter in the range of 6.0 mm to 8.0 mm with 7.0 mm being the preferred diameter.

Referring to Figures 15A, 15B, and 15C, an enlarged cross sectional view of three different embodiments of a securing means 65 for locking the screw 60 once it is threaded to the spinal fusion, implant 40 are shown. In Figure 15A, the securing means 65 comprises a notch 66 in the surface 20 of the top member 14 which is preferably made of metal. Once the screw 60 is threaded and securely tightened to the spinal fusion implant 40, a chisel C is used to bend a portion 67 of the top member 14 into the central opening 18 and against the screw head 64 so as to prevent the outward excursion and any unwanted loosening of the screw 60.

In Figure 15B, a second embodiment of the securing means 65a is shown comprising a central score 66a concentric with the central opening 18. A screw 60a having a slot 61a in the screw head 64a is threaded and securely tightened to the spinal fusion implant 40. An instrument T is partially inserted into slot. 61a after which an impaction force F₁ is applied to the instrument T to spread apart the screw head 64a in the direction of the arrows A so that the screw head 64a becomes deformed from the impaction force F₁ and fits within the central score 66a. Once the screw head 64a is in the central score 66a, the outward excursion of the screw 60a is prevented by the top lip 68 of the central score 66a.

In Figure 15C, a third embodiment of the securing means 65b is shown comprising a screw 60b having a screw head 64b with a slightly flanged portion 69b near the top and a slot 61b. The central opening 18 has along its circumference a recess 66b for receiving the flanged portion 69b of the screw head 64b. The securing means 65b relies on the natural resiliency of the metal screw head 64b such that when the screw 60b is being driven by a screw driver, the screw head 64b flexes in the direction of the arrows B. In this manner, the flanged portion 69b of the screw head 64b slides along the interior of the central opening 18 so that the screw head 64b is below the top lip 68b of the recess 66b. Once the screw driver is removed from the screw 60b, the screw head 64b returns to its natural state in the direction opposite to the arrows B so that the flanged portion 69b is within the recess 66b. The outward excursion of the screw 60 is thus prevented by the top lip 68b which blocks the screw head 64b by catching the flanged portion 69b.

Figures 16A-18 show the instrumentation used for installing the spinal fixation device 10. Referring to Figure a driving instrument 80 used for inserting the spinal fixation device 10 into the vertebrae V is shown having a hollow tubular shaft 82 which terminates at one end to a bottom flat member 84 and terminates to a top flat member 86 at the other end. The bottom flat member 84 is preferably configured so that it conforms to the shape of the top member 14 of the staple member 12.

The driving instrument 80 has a pair of short posts 88a and 88b extending from the bottom flat member 84. The posts 88a and 88b are oriented on the bottom flat member 84 so as to correspond to the position of the openings 22a and 22b in the upper surface 20 of the top member 14 of the staple member 12. Each of the posts 88a and 88b fit into each of the openings 22a and 22b and keep the staple member 12 aligned on the bottom f lat member 84 of the driving instrument 80. It is appreciated that the openings 22a and 22b in the top member 14 may be depressions within the surface 20 of the top member 14 or may be holes that pass through the top member 14. In the preferred embodiment, the openings 22a and 22b gave a diameter ranging from 1.5 mm to 3.5 mm, with 2.5 mm being the preferred diameter.

Referring to Figure 16B, an alternative embodiment of the driving instrument 80' which is used for inserting into the vertebrae V the spinal fixation device 210, described in detail below in reference to Figure 26, is shown having a hollow tubular shaft 82' which terminates at once end to a bottom flat member 84' and terminates to a top flat member 86' at the other end. The bottom flat member 84' is rectangular in shape so that it conforms to the shape of the top member 214 of the spinal fixation device 210.

The driving instrument 80' has a pair of short posts 88'a, 88'b, 88'c and 88'd extending from the bottom flat member 84'. The posts 88'a-88'd are oriented on the bottom flat member 84' so as to correspond to the position of the openings 222a-222d of the spinal fixation device 210. Each of the and keep the spinal fixation device 210 aligned on the bottom flat member 84' of the driving instrument 80'.

Referring to Figure 17A, an alignment rod 70 comprising a cylindrical shaft 72 having a smooth exterior surface 73 and a threaded end 74 may be threadably attached to the threaded aperture 45 of the spinal fusion implant 40 is shown. The alignment rod 70 fits through the central opening 18 of the spinal fixation device 10 and is used to properly align the projections 16 and 17 on each side of the spinal fusion implant 40 prior to engaging the vertebrae V. Further, the alignment rod 70 also serves as a guide post for the drilling template instrument 50 described in greater detail below.

Referring to Figure 17B, as an alternative embodiment of the alignment rod 70, a splined alignment rod 70' that has a finely splined surface 72' along its longitudinal axis and a threaded end 74' that may be attached to the threaded aperture 55 of the spinal fusion implant is shown.

Referring to Figure 18, a drilling template instrument 50 for creating a pair of insertion holes 53a and 53b in each of the vertebrae V for receiving each of the projection 16 and 17 respectively is shown. The drilling template instrument 50 has a template 52 with a central aperture 54 therethrough and guide passages 55 and 56 for guiding a drill bit 51 of a drilling tool. Attached to the template 52 is a handle 58 which angles away from the template 52 so as not to obstruct the line of sight of the surgeon and to allow easy access to the template 52 and easy access to the guide holes 55 and 56 for the drill bit 51. Extending from the center of the bottom surface of the template 52 is a central member 59 (similar in structure and function to the central bar 35) for mating to an already implanted intervertebral spinal fusion implant 40. The central member 59 interdigitates with the depression 42 of the spinal fusion implant 40 so that the template 52 is properly oriented about the spinal fusion implant 40 and the guide holes 55 and 56 are properly oriented with respect to the vertebrae V adjacent to the spinal fusion implant 40. The alignment rod 70 serves as a guide post for the drill template instrument 50 as it fits through the central aperture 54 of the template 52 and aligns the template 52 with respect to the spinal fusion implant 40 and insures that it is coaxial. The central aperture 54 of the drilling template instrument 50 is smooth so that if it is placed over a splined alignment rod 70' the drilling template instrument 50 may be easily rotated about the splined-alignment rod 70' into position such that the central member 59 is able to mate and interdigitate with the depression 44 of the spinal fusion implant 40.

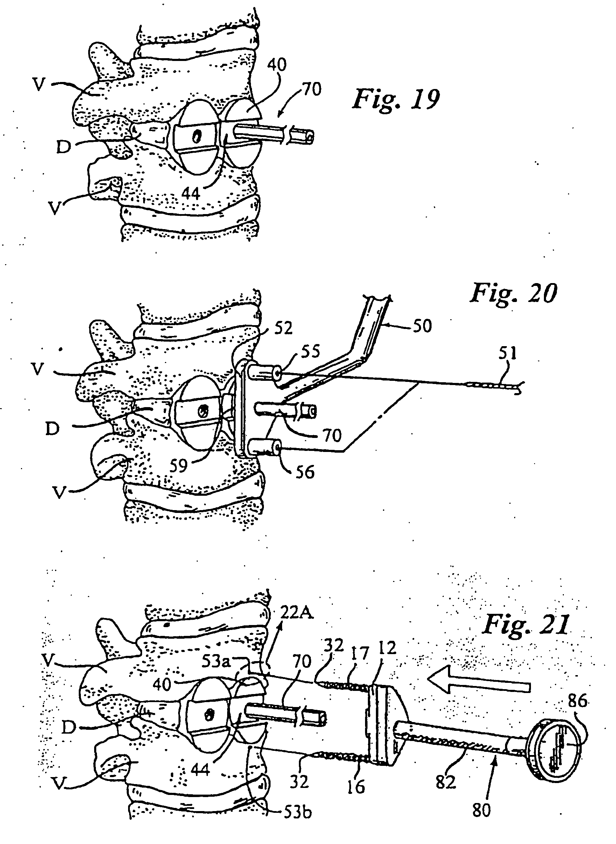

Referring to Figures 19-24, the spinal fixation device 10 is inserted in the following manner: At least one spinal fusion implant 40 is surgically implanted so that it is substantially within the disc space between two adjacent vertebrae V and engages at least a portion of each of the two adjacent vertebrae V. Once the spinal fusion implant 40 is in place, the alignment rod 70 is attached to the threaded aperture 45 of the spinal fusion implant 40. The alignment rod 70 serves as a guide post for the drilling template instrument 50 as it fits through the central aperture 54 of the template 52 and aligns the template 52 coaxially with respect to the spinal fusion implant 40. Referring to Figure 20, once the template 52 is properly aligned and the drilling template instrument 50 is seated so that the central member 59 interdigitates with the spinal fusion implant 40, the insertion holes 53a and 53b are drilled in each of the adjacent vertebrae V with a drilling instrument having a drill bit 51 with a diameter that is substantially smaller than the diameter of each the projections 16 and 17 of the staple member 12.

Once the drilling of the insertion holes 53a and 53b is completed, the drill template instrument 50 is removed from the spinal fusion implant 40 and from the alignment rod 70. The alignment rod 70 is left in place attached to the threaded aperture 45 of the spinal fusion implant 40.

Referring to Figure 21, the staple member 12 is placed onto the driving instrument 80 used for driving and fixing the staple member 12 into the vertebrae V so that the bottom flat member 84 and the posts 88a and 88b are aligned with the top member 14 and the depressions 22a and 22b of the top member 14. The alignment rod 70 serves as a guide post for the staple member 12 as it fits through the central opening 18 of the staple member 12 and aligns the staple member 12 coaxially with respect to the spinal fusion implant 40.

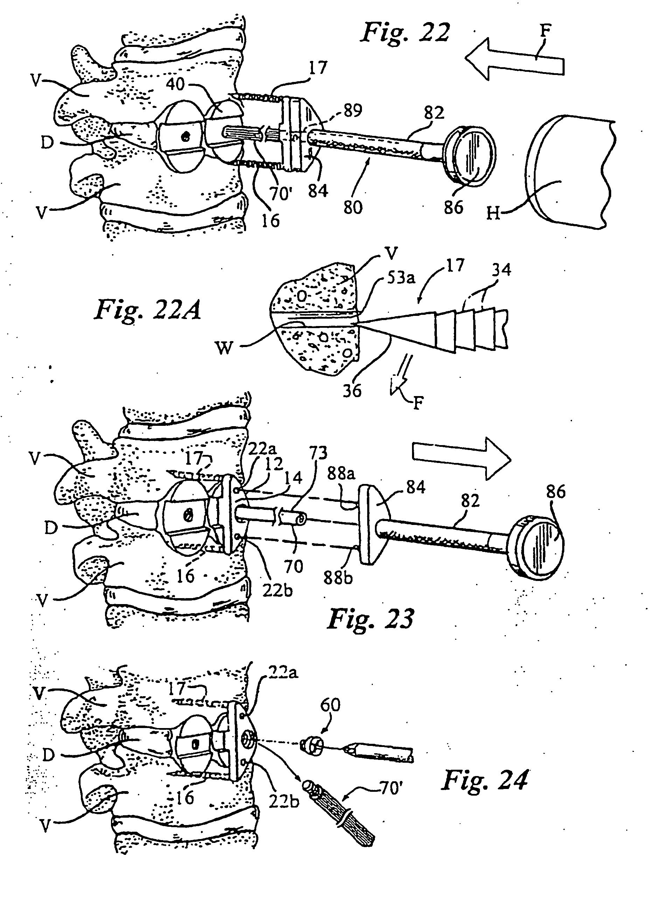

Referring to Figure 22, once the staple member 12 is properly placed onto the bottom flat member 84 of the driving instrument 80, the staple member 12 and the driving instrument 80 are aligned with respect to the alignment rod 70 so that the alignment rod 70 passes through the central opening 18 of the staple member 12 and is inserted into the central hollow portion 89 of the driving instrument 80. The staple member 12 and the driving instrument 80 are then lowered along the alignment rod 70 so that the sharp distal end 32 of each of the projections 16 and 17 comes into contact with the external surface of the vertebrae V and is aligned with the previously drilled insertion holes 53.

As shown in Figure 22A, it is preferred that the insertion holes 53a and 53b be drilled so that when the projections 16 and 17 are inserted into the holes 53a and 53b, the incline planed inner surface 36 of each of the projections 16 and 17 contacts the inner wall W of the insertion holes 53a and 53b that is closest to the spinal fusion implant 40. In this manner a compression force F is created as each of the projections 16 and 17 of the staple member 12 is inverted into insertion holes 53a and 53b, respectively, compressing the bone of the vertebrae V toward the spinal fusion implant 40.

Referring to Figure 23, the staple member 12 is then driven into the vertebrae V by applying a high impaction force to the driving instrument 80 with a hammer H or other impacting means against the top flat member 86 of the driving instrument 80. The staple member 12 is driven into the vertebrae V such that the projections 16 and 17 are moved forward into the insertion holes 53a and 53b, respectively, until the bottom surface 30 of the top member 14 of the staple member 12 comes to rest against the surface of the vertebrae V.

Referring to Figures 23-24, the driving instrument 80 is lifted away from the alignment rod 70 so that the alignment rod 70 is no longer within the central hollow portion 89 of the driving instrument 80. The alignment rod 70. is unthreaded from the threaded aperture 45 and is removed from the spinal fusion implant 40. The staple member 12'is secured to the spinal fusion implant 40 with the locking screw 60 which has a threaded, pattern 62 with a reduced pitch. The reduced pitch of the locking screw 60 locks the locking screw 60 to the spinal fusion implant 40 with minimal turning of the locking screw 60 and prevents any unwanted loosening. Further, any of the three embodiments of the securing means 65, 65a or 65b described above in reference to Figures 15A-15C may be used to further prevent any unwanted loosening and outward excursion of the screw 60.

Referring back to Figure 12, once the staple member 12 is driven into the vertebrae V and is secured to the spinal fusion implant 40, the spinal fusion implant 40 is prevented from rotating along its rotational axis R by its connection to the staple member 12 which is fixated across the disc space between the vertebrae V. The staple member 12 is prevented from backing out from the vertebrae V along the longitudinal axis L by its connection to the spinal fusion implant 40 and by the segmented and ratcheted portions 34 of the projections 16 and 17. In this manner, the staple member 12 and the spinal fusion implant 40 interact to prevent the dislodgement of each other from the vertebrae V in which they are implanted. Thus, the staple member 12 is made safe against dislodgement by attachment to the spinal fusion implant 40 and the stability of the spinal fusion implant 40 is assured as it is also stabilized by the staple member 12 and each works in connection with the other to remove the only remaining degree of freedom that would allow for the disengagement of either. In addition, the incline planed inner surface 36 at the distal end 32 of the projections 16 and 17 forces bone toward the spinal, fusion implant 40 along force lines F to further secure the spinal fusion implant 40 and further prevent the dislodgement of the spinal fusion implant 40.

It is appreciated by those skilled in the art that when the bone of the vertebrae V is sufficiently soft, a shorter method (hereinafter referred to as the "Short Method") of inserting the spinal fixation device 10 is possible by omitting the steps of drilling the insertion holes 53a and 53b prior to inserting the staple member 12 into the vertebrae V.

Referring to Figure 21, in the Short Method, the splined alignment rod 70' that is finely splined along its longitudinal axis is used instead of the alignment rod 70. Once the splined alignment rod 70' has been attached to the spinal fusion implant 40, the staple member 12 may be placed over the splined alignment rod 70', so that the splined alignment rod 70' passes through the aperture 18 and into the central aperture 89 of the driving instrument 80. The central aperture 89 of the driving instrument 80 is correspondingly splined to the splines of the splined alignment rod 70' so that the staple member 12 can be aligned with respect to the spinal implant 40. The alignment of the staple member 12 and the driving instrument 80 is maintained as the corresponding splines of the central aperture 89 interdigitate with the splines of the splined alignment rod 70' and prevent the rotation of the staple member 12 about the splined alignment rod 70'. The prevention of rotation about the splined alignment rod 70' is especially important when the Short Method is used to insert the spinal fixation device 10, as no insertion holes 53a and 53b have been drilled in the vertebrae V. The staple 12 can be driven directly into the vertebrae V by the application of a high impaction force to the driving instrument 80 as described above and shown in Figure 32.

Once the staple member 12 is driven into the vertebrae V, the steps of the longer method described above are used to secure the spinal fixation device to the spinal fusion implant 40 are the same. The Short Method of inserting the staple member 12 reduces the amount of time required to insert and secure the spinal fixation device 10 of the present invention and thus reduces the overall duration of the spinal fixation surgical procedure.

Referring to Figure 27, an apparatus having a spinal fixation device 310 according to the invention is shown, having a staple 312 with a top member 314 that is generally triangular is shown. The top member 314 has two projections 316 and 317 depending from the bottom surface of the top member 314 that engage the vertebrae V. Expending from the center of the bottom surface of the top member 314 is a central member 390 which is similar to the central bar 35 of the preferred embodiment of the spinal fixation device 10 in that the central-member 390 interdigitates with the depression 44a of the spinal fusion implant 40. However, the central bar 390 also has an extension arm 392 that extends laterally from the top member 314 to span the diameter of an adjacent spinal fusion implant 41. The extension arm 392 interdigitates with the depression 44 of the spinal implant 41. The extension arm 392 has a central aperture 374 for receiving a screw 60b used to couple the extension arm 392 to the spinal fusion implant 41. In this manner, a single spinal fixation device 310 is capable of interdigitate with two adjacent spinal fusion implants 40 and 41 to lock and prevent the rotation and any excursion of the spinal fusion implants 40 and 41. The fixation of two spinal fusion implants 40 and 41 is possible while leaving no protruding metal, such as the top member 314, on the side of the spine where the vessels are located in' close approximation to the vertebrae as is the case with the L₄ and L₃ vertebrae where the vessels are located over the left side of those vertebrae. It is appreciated that any of the securing means 65-65b, described above may be used to lock the screw 60b to the extension arm 392.

Alternatively, for all of the examples described above, the spinal fixation device 10 could be made of resorbable materials, such as bio-compatible resorbable plastics, that resorb at an appropriate rate such that once the spinal fixation device 10 is no longer needed (i.e. when spiral fusion is complete) the body would resorb the spinal fixation device 10. One such resorbable material is polygalactone, however any other resorbable plastic or other material safely usable within the human body are also within the scope of the present invention.

Further, the spinal fixation device could be only in part resorbable such that the projections 16 and 17 of the staple member 12 would be non-resorbable and would remain incarcerated in the vertebrae V and sealed off once the resorbable portion of the staple is resorbed by the body.

While the present invention has been described with respect to its preferred embodiment and a number of alternative embodiments, it is recognized that additional variations of the present invention may be devised without departing from the scope as defined by the claims.

## Claims

1. An apparatus for providing linked multiple spinal implants, comprising:
a first spinal implant (40) adapted to be surgically implanted at least in part within a disc space between two adjacent vertebrae in a segment of the spine, said first spinal implant (40) being adapted to contact both of the vertebrae adjacent to the disc space;
a second spinal implant (41) adapted to be surgically implanted at least in part within the same disc space in which said first spinal implant (40) is to be implanted, said second spinal implant (41) being adapted to contact both of the vertebrae adjacent to the disc space; and **characterised by** further comprising a spinal fixation device (310) in the form of a staple (312) with a top member (314) having interdigitating means (390, 192) adapted to interdigitate with corresponding interdigitating means (44a, 44) provided on an end of each of said first and second spinal implants (40, 41) for connecting said first and second spinal implants (40, 41), and at least two projections (316, 317) extending from said top member (314) and adapted to be inserted into both of the vertebrae adjacent to the disc space.

2. The apparatus of any one of the above claims, wherein said first and second spinal implants (40, 41) further comprise a protrusion for engaging the adjacent vertebrae.

3. The apparatus of claim 2, wherein said protrusion is a thread.

4. The apparatus of any one of the above claims, wherein said interdigitating means (390, 392) is a bar, and said corresponding interdigitating means are depressions (44, 44a).

5. The apparatus of any one of the above claims, in combination with a fusion promoting material to facilitate the bone fusion process.

6. The combination of claim 5, wherein said fusion promoting material includes at least one of hydroxyapatite, hydroxyapatite tricalcium phosphate, and bone morphogenic protein.

## Patentansprüche

1. Gerät zum Bereitstellen verketteter multipler Rückenimplantate, umfassend:
ein erstes Rückenimplantat (40), das dazu geeignet ist, chirurgisch mindestens teilweise in einem Bandscheibenzwischenraum zwischen zwei nebeneinanderliegenden Wirbeln in einem Abschnitt der Wirbelsäule implantiert zu werden, wobei das erste Rückenimplantat (40) dazu geeignet ist, die beiden Wirbel neben dem Bandscheibenzwischenraum zu berühren;
ein zweites Rückenimplantat (41), das dazu geeignet ist, chirurgisch mindestens teilweise in demselben Bandscheibenzwischenraum implantiert zu werden, in den das erste Rückenimplantat (40) implantiert werden soll, wobei das zweite Rückenimplantat (41) dazu geeignet ist, die beiden Wirbel neben dem Bandscheibenzwischenraum zu berühren; und
**dadurch gekennzeichnet, dass** es ferner ein Rückenbefestigungselement (310) in Form einer Klammer (312) mit einem oberen Element (314), das fingerförmig ineinandergreifende Mittel (390, 192) aufweist, die dazu geeignet sind, mit entsprechenden fingerförmig ineinandergreifenden Mitteln (44a, 44), die an einem Ende von jedem der ersten und zweiten Rückenimplantate (40, 41) fingerförmig ineinanderzugreifen, um die ersten und zweiten Rückenimplantate (40, 41) und mindestens zwei Vorsprünge (316, 317), die sich von dem oberen Element (314) aus erstrecken und dazu geeignet sind, um in jeden der beiden Wirbel neben dem Bandscheibenzwischenraum eingefügt zu werden, zu verbinden, umfasst.

2. Gerät nach einem der vorhergehenden Ansprüche, wobei die ersten und zweiten Rückenimplantate (40, 41) ferner einen Vorsprung umfassen, um in die nebeneinanderliegenden Wirbel einzugreifen.

3. Gerät nach Anspruch 2, wobei der Vorsprung ein Gewinde ist.

4. Gerät nach einem der vorhergehenden Ansprüche, wobei die fingerförmig ineinandergreifenden Mittel (390, 392) eine Stange und die entsprechenden fingerförmig ineinandergreifenden Mittel Vertiefungen (44, 44a) sind.

5. Gerät nach einem der vorhergehenden Ansprüche, in Kombination mit einem verschmelzungsfördernden Material, um den Knochenverschmelzungsprozess zu erleichtern.

6. Kombination aus Anspruch 5, wobei das verschmelzungsfördernde Material mindestens eines von Hydroxylapatit, Hydroxylapatit-Trikalziumphosphat und knochenmorphogenen Protein umfasst.

## Revendications

1. Appareil pour fournir de multiples implants rachidiens liés, comprenant :
un premier implant rachidien (40) adapté de sorte qu'il soit implantable par chirurgie, au moins partiellement, dans un espace intervertébral entre deux vertèbres adjacentes dans un segment de la colonne vertébrale, ledit premier implant rachidien (40) étant adapté pour entrer en prise avec les deux vertèbres adjacentes à l'espace intervertébral;
un deuxième implant rachidien (41) adapté de sorte qu'il soit implantable par chirurgie, au moins partiellement, dans le même espace intervertébral dans lequel ledit premier implant rachidien (40) doit être implanté, ledit deuxième implant rachidien (41) étant adapté pour entrer en prise avec les deux vertèbres adjacentes à l'espace intervertébral; et **caractérisé en ce qu'**il comprend également un dispositif de fixation de vertèbre (310) sous forme d'une agrafe (312) ayant un membre supérieur (314) avec des moyens d'interdigitation (390, 392) adaptés pour interdigiter avec les moyens d'interdigitation correspondant (44a, 44) fournis sur une extrémité de chacun desdits premier et deuxième implants rachidiens (40, 41) pour connecter lesdits premier et deuxième implants rachidiens (40, 41), et au moins deux projections (316, 317) s'étendant du membre supérieur (314) et adapté pour être inséré dans les deux vertèbres adjacentes à l'espace intervertébral.

2. Appareil de l'une quelconque des revendications ci-dessus, dans lequel lesdits premier et deuxième implants rachidiens (40, 41) comprennent également une protubérance pour entrer en contact avec la vertèbre adjacente.

3. Appareil de la revendication 2, dans lequel ladite protubérance est un fil.

4. Appareil de l'une quelconque des revendications ci-dessus, dans lequel lesdits moyens d'interdigitation (390, 392) est une tige, et lesdits moyens d'interdigitation sont des creux (44, 44a).

5. Appareil de l'une quelconque des revendications ci-dessus, en association avec un matériau favorisant la fusion pour faciliter le processus de fusion des os.

6. Association de la revendication 5, dans lequel ledit matériau favorisant la fusion comprend au moins l'un de l'hydroxyapatite, le phosphate tricalcique hydroxyapatite et la protéine morphogénique de l'os.
